**Europäisches Patentamt**

**European Patent Office**

**Office européen des brevets**

(11) Veröffentlichungsnummer : **0 031 044**
**B1**

(12) # EUROPÄISCHE PATENTSCHRIFT

(45) Veröffentlichungstag der Patentschrift :
05.10.83

(21) Anmeldenummer : **80107402.2**

(22) Anmeldetag : **27.11.80**

(51) Int. Cl.³ : **C 07 C 69/67**, C 07 C 67/343

(54) **Verfahren zur Herstellung von Alkaliformylessigsäureester.**

(30) Priorität : 22.12.79 DE 2952070

(43) Veröffentlichungstag der Anmeldung :
01.07.81 Patentblatt 81/26

(45) Bekanntmachung des Hinweises auf die Patenterteilung : 05.10.83 Patentblatt 83/40

(84) Benannte Vertragsstaaten :
**BE DE FR GB IT NL**

(56) Entgegenhaltungen :
**DE C 708 513**
**FR A 2 128 410**

(73) Patentinhaber : **DYNAMIT NOBEL AKTIENGESELL-SCHAFT**
**Patentabteilung Postfach 1209**
**D-5210 Troisdorf, Bez. Köln (DE)**

(72) Erfinder : **El-Chahawi, Moustafa, Dr.**
**Pacellistrasse 4**
**D-521 Troisdorf (DE)**

## Verfahren zur Herstellung von Alkaliformylessigsäureester

Die vorliegende Erfindung betrifft ein Verfahren zur verbesserten Herstellung von Alkaliformylessigsäureestern aus Essigsäureester, Alkalialkoholat und Kohlenmonoxid unter Verwendung von erhöhter Temperatur und erhöhtem Druck.

Aus der US-PS 2 394 255 ist eine Umsetzung von Essigsäureester mit Alkalialkoholat in Gegenwart von Alkylformiat oder Kohlenmonoxid bzw. bevorzugt Alkylformiat und Kohlenmonoxid bekannt. Dort wird ein Überschuß von Natriumalkoholat gegenüber Essigsäureester verwendet, wobei zusätzliche Mengen Alkylformiat bzw. Methanol anwesend ist, welches in Gegenwart von Kohlenmonoxid Alkylformiat bildet.

Als Reaktionsprodukt entsteht eine « slurry », d. h. eine Aufschlämmung fester Stoffe in überschüssigem Alkylformiat und Alkalialkoholat, welche schwer zu Alkali-formylessigester aufgearbeitet werden kann, so daß in der US-PS eine Reaktion dieser slurry zu einem Folgeprodukt ausgeführt wird.

Ausbeute und Reinheit sind daher unbefriedigend und die Verluste in Alkylformiat und Alkalialkoholat erheblich. Nach der DE-PS 708 513 wird Alkaliäthylat und Äthylacetat im Molverhältnis 1 : 1 in Gegenwart von großen Mengen Alkohol als Lösungsmittel mit CO umgesetzt. Es werden 85 % Ausbeute einer Substanz von nicht genannter Reinheit erhalten, die sich zum Oxymethylenessigsäureäthylester nur zu 17 % umsetzen ließ.

Es wurde nun gefunden, daß hohe Ausbeuten und Reinheiten von Alkaliformylessigsäurealkylester erzielt werden, wenn als Reaktionsprodukte lediglich der Essigsäurealkylester, Alkalialkoholat und Kohlenmonoxid anwesend ist und auf die vorherige Bildung und die Verwendung von Alkylformiat verzichtet wird. Essigsäureester soll im Überschuß zu Alkalialkoholat vorliegen.

Gegenstand der Erfindung ist daher ein Verfahren zur Herstellung von Alkaliformylessigsäurealkylester durch Umsetzung von Essigsäurealkylester mit Alkalialkoholat und Kohlenmonoxid bei erhöhtem Druck und erhöhter Temperatur, welches dadurch gekennzeichnet ist, daß ein Überschuß von mindestens 1,2 Mol Essigsäurealkylester, bezogen auf Alkalialkoholat, in Abwesenheit von Lösungsmitteln umgesetzt wird.

Ganz wesentlich ist die Verwendung eines Überschusses von Essigester gegenüber Alkalialkoholat, wobei mindestens 1,2 Mol Essigester je Mol Alkalialkoholat anwesend sind und bevorzugt 2 bis 4 Mole Essigester je Mol Alkalialkoholat verwendet werden, wobei auch ein Überschuß von Essigester bis zu 6 Mol gegebenenfalls auch mehr, möglich ist.

Kohlenmonoxid soll in Mengen von mindestens 1 Mol je Mol Alkalialkoholat verwendet werden. Kohlenmonoxid kann bei der Reaktion inerte Gase, beispielsweise Stickstoff, enthalten. Der Reaktionsdruck soll 1 bis 100 bar, bevorzugt von 10 bis 50 bar, betragen. Die Reaktionstemperatur kann zwischen 10 und 100 °C, vorzugsweise zwischen 30 und 80 °C liegen.

Unter diesen Bedingungen kann die Reaktion in einer Stunde oder weniger vollständig ablaufen und Ausbeuten von 90 Gew.-% und darüber erreichen.

Als Essigester wird sehr bevorzugt Essigsäureäthylester und ggf. Essigsäuremethylester verwendet.

Als Alkalialkoholat ist Natriumäthylat sehr bevorzugt und Natriummethylat bzw. Kaliumäthylat bzw. -methylat möglich.

Das verwendete Akalialkoholat kann 85 bis 97 %ig sein, wobei vorzugsweise das technische 95 %ige Produkt zum Einsatz kommt, was einen kleinen Unterschuß von Alkali aufweist. Es erwies sich als sehr vorteilhaft, daß nunmehr Alkaliformylessigester durch Kristallisation als reine Substanz aus dem Reaktionsgemisch gewonnen und sehr gut von überschüssigem Essigsäureester durch Filtration getrennt werden kann. Anhaftender Essigsäureester kann leicht im Vakuum oder durch Temperaturerhöhung entfernt werden. Der überschüssige Essigester wird dem nächsten Ansatz zugefügt, so daß praktisch keine Stoffverluste oder zu beseitigende Abstoffe auftreten.

Es erwies sich, daß die Abwesenheit von Lösungsmitteln nach der Erfindung die Rückführung des überschüssigen Essigesters und dessen Umsetzung erheblich erleichtert. Es ist überraschend, daß die Ausführung der Reaktion in Abwesenheit der nach dem Stand der Technik verwendeten großen Mengen von Lösungsmitteln möglich ist und in kurzer Zeit mit hohen Ausbeuten verläuft.

Weiter ist überraschend, daß eine Abwesenheit von Alkohol in freier Form möglich ist, da unter diesen Bedingungen Essigester mit Natriumalkoholat nach der bekannten Claisen-Kondensation Acetessigester bilden müßte.

Die hergestellten Stoffe dienen als Zwischenprodukte für u. a. die pharmazeutische Industrie.

Beispiel 1

In einem mit einem Rührer und Gaseinleitungsrohr versehenen Druckkessel von 250 l Inhalt werden in Stickstoffatmosphäre 37 kg (514 Mol) 95 Gew.-%ig Natriumäthylat und 154 kg (1 759 Mol) Essigsäureäthylester vorgelegt. Der Kessel wird mit CO gespült. Kohlenmonoxid wird unter Rühren bis zu einem Druck von 20 bar zugefügt und die Temperatur auf 80 °C erhöht. Bei dem Aufheizen erfolgt eine starke Aufnahme von CO und mit weiterem CO wird der Druck von 20 bar aufrechterhalten. Nach 1 Stunde ist die Reaktion beendet. Der Autoklav wird gekühlt und von überschüssigem CO befreit. Nach Spülen mit Stickstoff wird das feste Produkt mit einer Schleuderzentrifuge abgetrennt. Man erhält 72,5 kg Natriumformylessigsäureäthylester. Nach Entfernung von anhaftendem Essigester wurden 64 kg erhalten. Ausbeute 90 %.

Die Umsetzung zum Semicarbazon ergab, daß der Natriumformylessigester eine Reinheit von über 95 % aufweist.

Beispiel 2

Das erhaltene Filtrat wurde mit einer kleinen Menge frischem Essigester ergänzt und mit Natriumäthylat und CO wie im Beispiel 1 versetzt. Die Reaktion war in 35 Minuten beendet und ergab die gleiche Ausbeute und Reinheit wie nach Beispiel 1.

**Ansprüche**

1. Verfahren zur Herstellung von Alkaliformylessigsäurealkylester durch Umsetzung von Essigsäurealkylester mit Alkalialkoholat und Kohlenmonoxid bei erhöhtem Druck und erhöhter Temperatur, dadurch gekennzeichnet, daß ein Überschuß von mindestens 1,2 Mol Essigsäurealkylester, bezogen auf Alkalialkoholat, in Abwesenheit von Lösungsmitteln umgesetzt wird.

2. Verfahren nach Anspruch 1, dadurch gekennzeichnet, daß bis zu 6 Mol Essigsäureester je Mol Natriumalkoholat verwendet werden.

3. Verfahren nach Anspruch 2, dadurch gekennzeichnet, daß der Überschuß an Essigsäureester 2 bis 4 Mol je Mol Alkalialkoholat beträgt.

4. Verfahren nach einem der Ansprüche 1 bis 3, dadurch gekennzeichnet, daß die Temperatur zwischen 10 und 100 °C liegt.

5. Verfahren nach einem der Ansprüche 1 bis 4, dadurch gekennzeichnet, daß der Druck 1 bis 100 bar beträgt.

6. Verfahren nach einem der Ansprüche 1 bis 5, dadurch gekennzeichnet, daß die Umsetzung mit Kohlenmonoxidhaltigem Gasgemisch durchgeführt wird.

**Claims**

1. Process for the preparation of alkali formyl acetic acid alkyl esters by reaction of acetic acid alkyl esters with alkali alcoholate and carbon monoxide at elevated pressure and elevated temperature, characterised in that an excess of at least 1.2 mole of acetic acid alkyl ester, related to alkali alcoholate, is reacted in the absence of solvents.

2. Process according to claim 1, characterised in that up to 6 mole of acetic acid ester are used per mole of sodium alcoholate.

3. Process according to claim 2, characterised in that the excess of acetic acid ester amounts to 2 to 4 mole per mole of alkali alcoholate.

4. Process according to one of claims 1 to 3, characterised in that the temperature lies between 10 and 100 °C.

5. Process according to one of claims 1 to 4, characterised in that the pressure amounts to 1 to 100 bar.

6. Process according to one of claims 1 to 5, characterised in that the reaction is carried out with carbon monoxide-containing gas mixture.

**Revendications**

1. Procédé de préparation d'esters d'acide formylacétique alcalin par réaction d'un acétate d'alkyle sur un alcoolate alcalin et du monoxyde de carbone sous pression et température élevées, caractérisé en ce que l'on fait réagir un excès d'au moins 1,2 mole d'acétate d'alkyle, par rapport à l'alcoolate de alcalin, en l'absence de solvants.

2. Procédé selon la revendication 1, caractérisé en ce que l'on utilise jusqu'à 6 moles d'acétate par mole d'alcoolate de sodium.

3. Procédé selon la revendication 2, caractérisé en ce que l'excès d'acétate est de 2 à 4 moles par mole d'alcoolate alcalin.

4. Procédé selon l'une quelconque des revendications 1 à 3, caractérisé en ce que la température est comprise entre 10 et 100 °C.

5. Procédé selon l'une quelconque des revendications 1 à 4, caractérisé en ce que la pression est de 1 à 100 bars.

6. Procédé selon l'une quelconque des revendications 1 à 5, caractérisé en ce que la réaction se déroule avec un mélange de gaz contenant du monoxyde de carbone.